# EUROPEAN PATENT SPECIFICATION

(11) **EP 3 285 691 B1**
(45) Date of publication and mention of the grant of the patent: **08.04.2020**
(21) Application number: 16717150.3
(22) Date of filing: 19.04.2016
(51) Int. Cl.: A61F 2/34, A61F 2/30

(54) **COTYLOID PROSTHESIS FOR HIPBONE PROSTHESES**
NAPFGELENKPROTHESE FÜR HÜFTBEINPROTHESEN
PROTHÈSE COTYLOÏDIENNE POUR PROTHÈSES D'OS ILIAQUE

(30) Priority: 20.04.2015 IT MI20150565
(43) Date of publication of application: 28.02.2018
(73) Proprietor: Adler Ortho S.p.a., 20032 Cormano (IT)
(72) Inventor: CREMASCOLI, Patrizio, 20091 Bresso (IT); CREMASCOLI, Davide, 20019 Settimo Milanese (IT); CREMASCOLI, Edgardo, 20129 Milano (IT); CHIRON, Philippe, Toulouse (FR); FERON, Franck, Betton (FR); RIBAS FERNANDEZ, Manuel, Barcelona (ES); GOVAERS, Kristoffel Jozef M, 2920 Kalmthout (BE); RHYS EVANS, Aled, Cardiff South Glamorgan CF15 9 SY (GB)
(74) Representative: Modiano, Micaela Nadia
(86) International application number: PCT/EP2016/058609
(87) International publication number: WO 2016/169913

(56) References cited:
- EP-A2- 1 683 593
- EP-A2- 1 800 700
- WO-A2-2008/146141
- US-A- 3 903 549

## Description

The present invention relates to a cotyloid prosthesis, also known as acetabular cup prosthesis, particularly useful and practical in orthopedic surgery procedures for a hipbone prosthesis, i.e., for the complete replacement of the hip joint with a prosthetic implant.

The procedure for prosthetic replacement of the hip is an effective biomechanical solution for severe joint disorders, such as for example arthritis, rheumatoid arthritis, cephalic necrosis, the outcomes of previous traumas (fracture and/or dislocation of the hip) and medial fracture of the femur. In particular, hip arthrosis (coxarthrosis) is the most frequent pathological event. Such orthopedic surgical procedure allows patients affected by these hipbone disorders to solve or alleviate significantly the pain, recovering a high quality of life.

Currently, a hip prosthesis is constituted by mechanical elements that functionally replace the physiological joint, in particular its acetabular component and its femoral component; these mechanical elements comprise a cotyloid prosthesis and a femoral prosthesis or shank made of metal, on the upper end of which, known as neck, a metal or ceramic head is inserted.

The cotyloid prosthesis and the femoral shank of a hip prosthesis of a known type can be fixed respectively to the iliac bone and to the femur of the patient by using acrylic cement (cemented prosthesis) or, as happens increasingly often, by interlocking mechanically under pressure, by means of the so-called press-fit method, the prosthetic elements in the appropriately prepared seat without using acrylic cement (non-cemented prosthesis).

The cotyloid prosthesis and the femoral shank of a non-cemented hip prosthesis of the known type are usually made of titanium alloy and have a shape and/or an external surface that are suitable to facilitate the growth of bone tissue around them (osteointegration), allowing their final stabilization.

In particular, it is known that a cotyloid prosthesis is constituted by an external element, commonly known as metal back, to be placed in contact with the cotyloid cavity or acetabulum of the iliac bone of the patient, which has a cavity in which an internal element, commonly known as insert, is arranged and accommodates the head of the femoral shank.

The metal back, as can be deduced easily from its name, is usually made of metallic material, while the insert is usually made of polyethylene, metallic material or ceramic. The metal back and the insert of a cotyloid prosthesis of the known type are usually mated by the orthopedic surgeon in the operating room, during the implantation procedure.

EP1683593 discloses a cotyloid as defined in the preamble of claim 1.

The aim of the present invention is to overcome the limitations of the background art described above, by providing a cotyloid prosthesis for hip bone prostheses that allows to obtain effects that are similar to those that can be obtained with known solutions, allowing to have a greater stability and reduced risk of dislocation of the hipbone prosthesis, as well as prolonged durability of such hipbone prosthesis, with a consequent improvement of the quality of life for the prosthetic patient.

Within this aim, an object of the present invention is to devise a cotyloid prosthesis for hipbone prostheses that allows to reduce the production of debris, i.e., detritus of the materials used for production, in the periarticular space.

Another object of the present invention is to provide a cotyloid prosthesis for hipbone prostheses that allows to obtain an optimum primary grip with the cotyloid cavity or acetabulum of the iliac bone of the prosthetic patient, in particular by implanting the prosthesis proper by means of the so-called press-fit technique.

A further object of the present invention is to devise a cotyloid prosthesis for hipbone prostheses that allows to minimize the thickness of the metal back, thus maximizing the thickness of the insert.

Moreover, an object of the present invention is to devise a cotyloid prosthesis for hipbone prostheses that does not require additional finishing work with traditional machines after its production, thus reducing production times.

Not least object of the present invention is to provide a cotyloid prosthesis for hipbone prostheses that is highly reliable, relatively simple to provide and at low costs.

This aim, as well as these and other objects that will become better apparent hereinafter, are achieved by a cotyloid prosthesis for hipbone prostheses, comprising a metal back and an insert, both having a substantially hemispherical shape, characterized in that said metal back is provided by using additive powder technology.

Further characteristics and advantages of the invention will become better apparent from the description of a preferred but not exclusive embodiment of the cotyloid prosthesis for hipbone prostheses according to the invention, illustrated by way of nonlimiting example in the accompanying drawings, wherein:

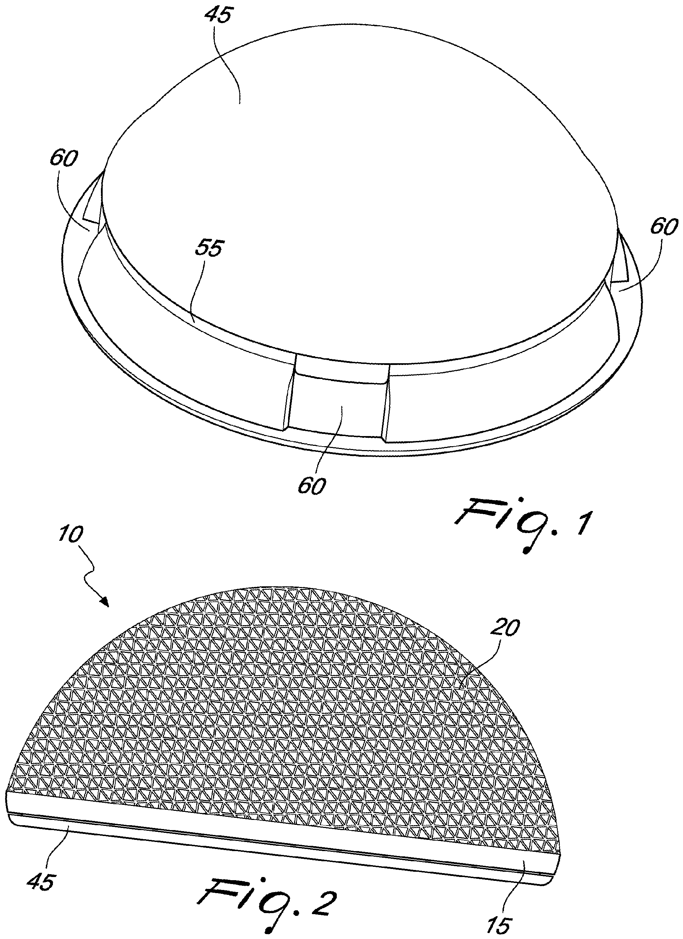

Figure 1 is a perspective view of the insert of an embodiment of a cotyloid prosthesis for hipbone prostheses according to the present invention;
Figure 2 is an elevation view of an embodiment of the cotyloid prosthesis for hipbone prostheses according to the present invention;

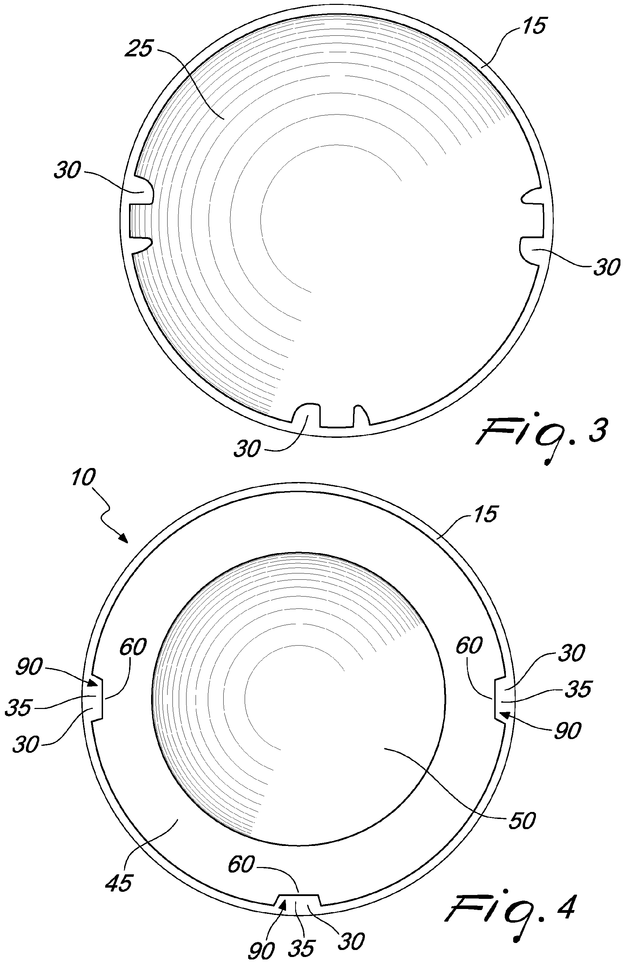

Figure 3 is a view of the lower face of the metal back of an embodiment of the cotyloid prosthesis for hipbone prostheses according to the present invention;
Figure 4 is a view of the lower face of an embodiment of the cotyloid prosthesis for hipbone prostheses according to the present invention;

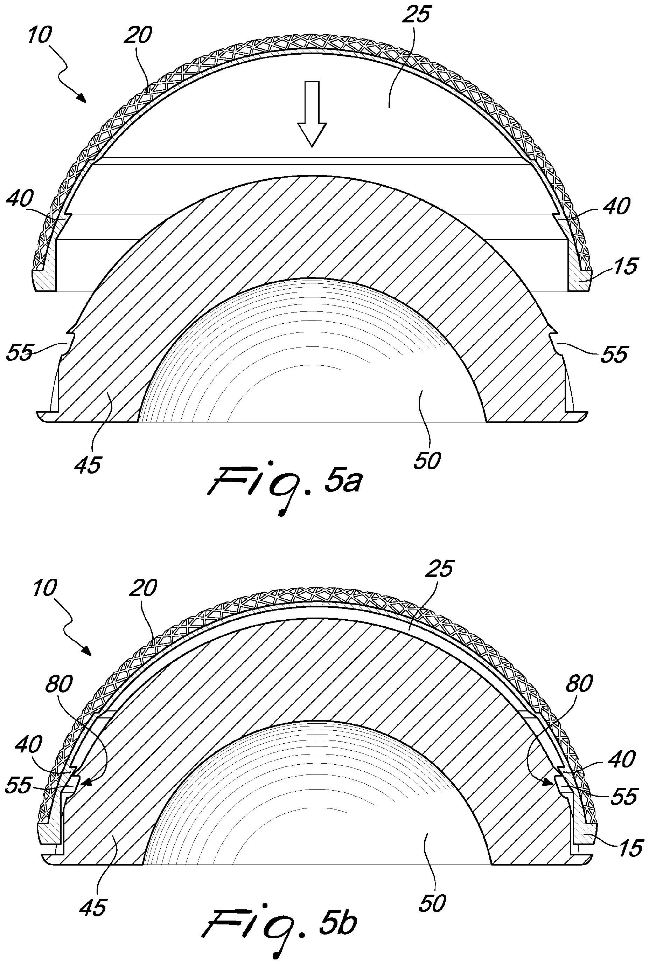

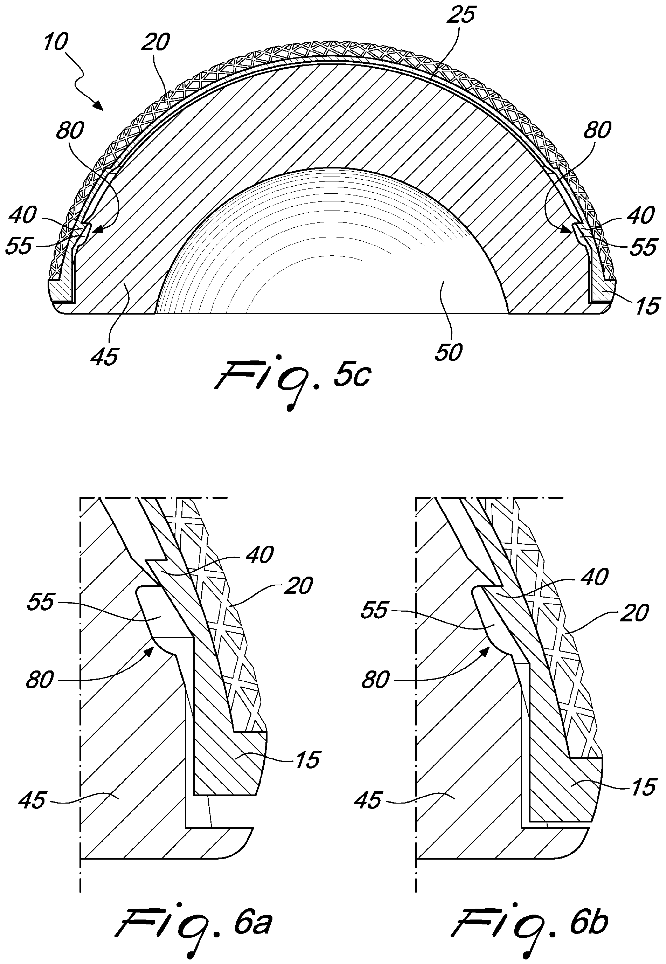

Figures 5a, 5b and 5c are sectional elevation views of an embodiment of the cotyloid prosthesis for hipbone prostheses according to the present invention, showing respectively three successive steps of the mating between the metal back and the insert by means of a coupling system;
Figures 6a and 6b are detail views of the system for coupling between the metal back and the insert comprised in the embodiment of the cotyloid prosthesis for hipbone prostheses according to the present invention, illustrated in Figures 5b and 5c.

With reference to the figures, the cotyloid prosthesis for hipbone prostheses according to the invention, designated generally by the referenced numeral 10, has a substantially hemispherical shape and comprises a metal back 15 and an insert 45, also having a substantially hemispherical shape; in particular, the metal back 15 is provided with a cavity 25 for coupling to the insert 45, while the insert 45 in turn is provided with a cavity 50 adapted to accommodate the head of a femoral shank.

The metal back 15 of the cotyloid prosthesis 10 is made of metallic material, preferably titanium alloy, even more preferably titanium alloy of the Ti6A14V type, and has a particularly low thickness, preferably comprised between 0.5 and 2 mm, even more preferably equal to 1.2 mm.

The metal back 15 is provided exclusively by additive production, i.e., by using exclusively additive powder technology, which allows to obtain, at the end of the production cycle, a high-precision finished product without the need to perform additional finishing work with traditional machines, thus reducing production times considerably.

Furthermore, by way of additive power technology it is possible to obtain calibrated porous lattices that have complex structures and also are made of metallic material, preferably titanium alloy.

In particular, the outer surface of the metal back 15 of the cotyloid prosthesis 10 has a layer of metal with a three-dimensional porous lattice 20, which has a thickness preferably comprised between 0.2 and 1 mm, even more preferably equal to 0.8 mm, and is constituted by numerous very sharp metallic cusps, which are particularly efficient in grip and accordingly ensure an optimum primary grip with the cotyloid cavity or acetabulum of the iliac bone of the prosthetic patient.

In a preferred embodiment of the cotyloid prosthesis 10 according to the invention, the metal cusps of the layer of porous metal 20 of the metal back 15 have a substantially triangular shape and are inclined at an acute angle, preferably comprised between 3° and 20°, even more preferably equal to 7°.

The insert 45 of the cotyloid prosthesis 10 is made of polyethylene with an ultra high molecular weight (UHMWPE - Ultra High Molecular Weight Polyethylene), with maximum thickness.

The choice of an insert 45 made of polyethylene, rather than metal or ceramic, leads to a reduction of the overall rigidity of the cotyloid prosthesis 10 that is to be implanted in the patient; this choice arises from the fact that the adoption of excessively rigid inserts can influence negatively the quality, particularly the bone mineral density, of the cotyloid cavity or acetabulum of the iliac bone of the prosthetic patient, due to the phenomenon known as "stress shielding", which can lead to osteolysis and/or mobilization of the cotyloid prosthesis, requiring an overhaul thereof.

In a preferred embodiment of the cotyloid prosthesis 10 according to the invention, the insert 45 can be made of UHMWPE polyethylene that is cross-linked and/or has an added antioxidant, preferably vitamin E or derivatives thereof.

In practice, the polyethylene insert 45 represents the articular interface with the head of the femoral shank, while the metal back 15 ensures an efficient primary grip at the interface with the cotyloid cavity or acetabulum of the iliac bone of the prosthetic patient, without phenomena of rotation or lever-out affecting the implanted cotyloid prosthesis 10.

The adoption of a metal back 15 that has a very low thickness entails the risk of possible deformations during the operations for implanting the cotyloid prosthesis 10 performed by the orthopedic surgeon in the operating room by means of the so-called press-fit technique.

For this reason, the cotyloid prosthesis 10 according to the invention is directly factory preassembled, by mating the metal back 15 and the insert 45 by means of a calibrated press, with a high level of quality that is ensured by an industrial control system.

By doing so, it is possible to provide the orthopedic surgeon with a preassembled cotyloid prosthesis that is very tough and already ready to be implanted in its seat, without any danger that it might be deformed during the press-fit operations.

Therefore, by way of the cotyloid prosthesis 10 according to the invention it is no longer the orthopedic surgeon who performs mating between the metal back 15 and insert 45, in the operating room during the implantation procedure, as usually occurs now.

The cotyloid prosthesis 10 according to the invention is manufactured in various sizes, in order to ensure the best quality of the product available to the orthopedic surgeon for the procedure for implantation in the patient. For example, the sizes available of the cotyloid prosthesis 10 according to the invention can vary between 40 mm and 70 mm, with a gap between one size and the next that is equal to 2 mm.

A preferred embodiment of the cotyloid prosthesis 10 for hipbone prostheses according to the invention further comprises a system 80 for coupling the insert 45 made of polyethylene in the cavity 25 of the metal back 15 made of titanium, so that the mating between the former 45 and the latter 15 ensures good resistance to torsion, to push-out and lever-out in the articulation with the head of the femoral shank.

The coupling system 80 is constituted by an annular protrusion or step 40 that is comprised in the metal back 15 and by an annular slot 55 comprised in the insert 45.

The annular protrusion or step 40 is provided on the inner surface of the metal back 15 and has a sharp edge that is oriented toward the cavity 25 of the metal back 15, being preferably arranged close to the base of the latter, approximately at one third of its height.

This sharp edge of the annular protrusion or step 40 engages the annular slot 55 provided on the outer surface of the insert 45, so as to ensure good mechanical strength of the mating between the metal back 15 and the insert 45 of the cotyloid prosthesis 10, under the typical mechanical stresses of the hipbone joint.

An even more preferred embodiment of the cotyloid prosthesis 10 for hipbone prostheses according to the invention comprises further a system 90 for interlocking the polyethylene insert 45 in the cavity 25 of the titanium metal back 15, in order to prevent rotational and torsional movements and further improve the mechanical strength of the mating between the metal back 15 and the insert 45 of the cotyloid prosthesis 10.

The interlocking system 90 is constituted by one or more rounded metal protrusions 30 that are comprised in the metal back 15 and enter perfectly, i.e., without play, seats 60 that are complementary thereto and are comprised in the insert 45.

In particular, the protrusions 30, preferably three in number, are provided along the inner circumference of the edge of the metal back 15 and are oriented toward the cavity 25 of the metal back 15, mutually spaced for example with a step of 90°. The seats 60 are instead provided along the outer circumference of the edge of the insert 45, in positions that are complementary to the positions of the protrusions 30.

In the preferred case in which the protrusions 30 are three in number, they are not distributed evenly along the inner circumference of the edge of the metal back 15 but are distributed only along the portion of such circumference that corresponds to the region of lowest load, so as to leave unchanged the thickness of the insert 45 in the region of maximum load, where the maximum thickness of UHMWPE polyethylene is required in order to ensure the best mechanical performance within the interaction between the insert 45 of the cotyloid prosthesis 10 and the head of the femoral shank.

The protrusions 30, in addition to their role within the interlocking system 90, also have a function of structural reinforcement of the metal back 15 and more generally of the cotyloid prosthesis 10 for hipbone prostheses.

In practice, the insert 45, once mated to the metal back 15 and coupled to the annular protrusion or step 40, is made to rotate so that the protrusions 30 comprised in the metal back 15 enter perfectly the seats 60 that are complementary thereto and are comprised in the insert 45, which thus remains locked integrally with the metal back 15 and thus is prevented from performing rotational and torsional movements as well as push-out and lever-out movements.

In an embodiment of the cotyloid prosthesis 10 for hipbone prostheses according to the invention, at the protrusions 30 comprised in the metal back 15 that belong to the interlocking system 90 it is possible to provide one or more coupling points 35 for the precision coupling of the so-called boneset or so-called extractor, which are instruments that are commonly used, the former for the procedure for implantation of a cotyloid prosthesis and the latter for the procedure for extracting a cotyloid prosthesis if an overhaul thereof is necessary.

In particular, the coupling points 35, preferably three in number, are provided along the inner circumference of the edge of the metal back 15, as mentioned at the protrusions 30 that belong to the interlocking system 90, and preferably are L-shaped; the boneset or extractor can couple, by means of a half rotation, with the coupling points 35 provided in the metal back 15, so that the connection between the boneset or the extractor and the cotyloid prosthesis 10 is solid and allows safe intervention on the part of the orthopedic surgeon.

In practice it has been found that the invention achieves fully the intended aim and objects. In particular, it has been shown that the cotyloid prosthesis for hipbone prostheses thus conceived allows to overcome the quality limitations of the background art, since it allows to have greater stability and reduced risk of dislocation of the hipbone prosthesis by enlarging the supporting surfaces, as well as a prolonged durability of the hipbone prosthesis proper, with a consequent improvement of the quality of life for the prosthetic patient.

Furthermore, the cotyloid prosthesis for hipbone prostheses according to the invention allows to reduce the production of debris in the periarticular space and allows to obtain an optimum primary grip with the cotyloid cavity or acetabulum of the iliac bone of the prosthetic patient.

A further advantage of the cotyloid prosthesis for hipbone prostheses according to the invention resides in that it allows to minimize the thickness of the metal back, thus maximizing the thickness of the insert and accordingly increasing the diameter of the head of the femoral shank to be articulated in the insert.

The invention thus conceived is susceptible of numerous modifications and variations, all of which are within the scope of the appended claims. All the details may further be replaced with other technically equivalent elements.

In practice, the materials used, as well as the contingent shapes and dimensions, may be any according to requirements and to the state of the art.

In conclusion, the scope of the protection of the claims must not be limited by the illustrations or preferred embodiments shown in the description by way of example, but rather the claims must comprise all the characteristics of patentable novelty that reside in the present invention, including all the characteristics that would be treated as equivalents by the person skilled in the art.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. A cotyloid prosthesis (10) for hipbone prostheses, comprising a metal back (15) and an insert (45), both substantially hemispherical in shape, said metal back (15) being provided by using additive powder technology, **characterized in that** it further comprises a rotation preventing interlocking system (90) for interlocking said insert (45) within said metal back (15), said rotation-preventing interlocking system (90) being constituted by one or more rounded metallic protrusions (30) defined along the inner circumference of the edge of said metal back (15), and by one or more seats (60) that are defined along the outer circumference of the edge of said insert (45) and are complementary to said one or more protrusions (30).

2. The cotyloid prosthesis (10) for hipbone prostheses according to claim 1, **characterized in that** said metal back (15) has a thickness comprised between 0.5 and 2 millimeters.

3. The cotyloid prosthesis (10) for hipbone prostheses according to claim 1 or 2, **characterized in that** the outer surface of said metal back (15) comprises a layer (20) of porous metal constituted by sharp metallic cusps.

4. The cotyloid prosthesis (10) for hipbone prostheses according to claim 3, **characterized in that** said layer (20) of porous metal has a thickness comprised between 0.2 and 1 millimeters.

5. The cotyloid prosthesis (10) for hipbone prostheses according to claim 3 or 4, **characterized in that** said cusps have a substantially triangular shape and an inclination comprised between 3° and 20°.

6. The cotyloid prosthesis (10) for hipbone prostheses according to one or more of the preceding claims, **characterized in that** it further comprises a system (80) for coupling said insert (45) in said metal back (15), said coupling system (80) being constituted by an annular step (40) defined on the inner surface of said metal back (15) and by an annular slot (55) defined on the outer surface of said insert (45).

7. The cotyloid prosthesis (10) for hipbone prostheses according to one or more of the preceding claims, **characterized in that** it further comprises one or more coupling points (35) for a boneset or extractor, said one or more coupling points (35) being defined at said protrusions (30).

8. The cotyloid prosthesis (10) for hipbone prostheses according to one or more of the preceding claims, **characterized in that** said metal back (15) is made of titanium alloy of the Ti6A14V type.

9. The cotyloid prosthesis (10) for hipbone prostheses according to one or more of the preceding claims, **characterized in that** said insert (45) is made of UHMWPE polyethylene.

10. The cotyloid prosthesis (10) for hipbone prostheses according to claim 9, **characterized in that** said insert (45) is made of UHMWPE polyethylene that is cross-linked and/or has an added antioxidant.

11. The cotyloid prosthesis (10) for hipbone prostheses according to claim 10, **characterized in that** said antioxidant comprises vitamin E or derivatives thereof.

12. The cotyloid prosthesis (10) for hipbone prostheses according to one or more of the preceding claims, **characterized in that** it is factory preassembled.

## Patentansprüche

1. Eine Napfgelenkprothese (10) für Hüftknochenprothesen, die eine Metallrückseite (15) und einen Einsatz (45), beide im Wesentlichen halbkugelförmig, umfasst; wobei die Metallrückseite (15) durch Additivpulver-Technologie hergestellt wird; **dadurch gekennzeichnet, dass** sie weiter ein rotationsverhinderndes Blockiersystem (90) zum Blockieren des Einsatzes (45) in der Metallrückseite (15) umfasst; wobei das rotationsverhindernde Blockiersystem (90) aus einem oder mehreren gerundeten Metallvorsprüngen (30), die entlang dem Innenumfang des Randes der Metallrückseite (15) bestimmt sind, und einem oder mehreren Sitzen (60) besteht, die entlang dem Außenumfang des Randes des Einsatzes (45) bestimmt und komplementär zu den einen oder mehreren Vorsprüngen (30) sind.

2. Die Napfgelenkprothese (10) für Hüftknochenprothesen gemäß Anspruch 1, **dadurch gekennzeichnet, dass** die Metallrückseite (15) eine Dicke zwischen 0,5 und 2 Millimetern hat.

3. Die Napfgelenkprothese (10) für Hüftknochenprothesen gemäß Anspruch 1 oder 2, **dadurch gekennzeichnet, dass** die Außenfläche der Metallrückseite (15) eine Schicht (20) von porösem Metall umfasst, die aus scharfen metallischen Spitzen besteht.

4. Die Napfgelenkprothese (10) für Hüftknochenprothesen gemäß Anspruch 3, **dadurch gekennzeichnet, dass** die Schicht (20) von porösem Metall eine Dicke zwischen 0,2 und 1 Millimeter hat.

5. Die Napfgelenkprothese (10) für Hüftknochenprothesen gemäß Anspruch 3 oder 4, **dadurch gekennzeichnet, dass** die Spitzen eine im Wesentlichen dreieckige Form und eine Neigung zwischen 3° und 20° haben.

6. Die Napfgelenkprothese (10) für Hüftknochenprothesen gemäß einem oder mehreren der obigen Ansprüche, **dadurch gekennzeichnet, dass** sie weiter ein System (80) zur Kopplung des Einsatzes (45) in der Metallrückseite (15) hat, wobei das Kopplungssystem (80) aus einer ringförmigen Stufe (40), die an der Innenfläche der Metallrückseite (15) bestimmt ist, und einem Kreisschlitz (55) besteht, der an der Außenseite des Einsatzes (45) bestimmt ist.

7. Die Napfgelenkprothese (10) für Hüftknochenprothesen gemäß einem oder mehreren der obigen Ansprüche, **dadurch gekennzeichnet, dass** sie weiter einen oder mehrere Kopplungspunkte (35) für einen Beinwell oder Extraktor umfasst, wobei die einen oder mehreren Kopplungspunkte (35) an den Vorsprüngen (30) bestimmt sind.

8. Die Napfgelenkprothese (10) für Hüftknochenprothesen gemäß einem oder mehreren der obigen Ansprüche, **dadurch gekennzeichnet, dass** die Metallrückseite (15) aus Titanlegierung vom Ti6A14V-Typ besteht.

9. Die Napfgelenkprothese (10) für Hüftknochenprothesen gemäß einem oder mehreren der obigen Ansprüche, **dadurch gekennzeichnet, dass** der Einsatz (45) aus UHMWPE-Polyethylen besteht.

10. Die Napfgelenkprothese (10) für Hüftknochenprothesen gemäß Anspruch 9, **dadurch gekennzeichnet, dass** der Einsatz (45) aus UHMWPE-Polyethylen besteht, das quervernetzt ist und/oder ein hinzugefügtes Antioxidans hat.

11. Die Napfgelenkprothese (10) für Hüftknochenprothesen gemäß Anspruch 10, **dadurch gekennzeichnet, dass** das Antioxidans Vitamin E oder Derivate davon umfasst.

12. Die Napfgelenkprothese (10) für Hüftknochenprothesen gemäß einem oder mehreren der obigen Ansprüche, **dadurch gekennzeichnet, dass** sie in der Fabrik vormontiert ist.

## Revendications

1. Prothèse cotyloïdienne (10) pour prothèses d'os iliaque, comportant un dos métallique (15) et un insert (45), tous deux ayant une forme sensiblement hémisphérique, ledit dos métallique (15) étant fourni en utilisant une technologie de poudres additives, **caractérisée en ce qu'**elle comporte en outre un système d'interverrouillage empêchant la rotation (90) pour in-terverrouiller ledit insert (45) à l'intérieur dudit dos métallique (15), ledit système d'interverrouillage empêchant la rotation (90) étant constitué d'une ou plusieurs protubérances métalliques arrondies (30) définies le long de la circonférence intérieure du bord dudit dos métallique (15), et d'un ou plusieurs logements (60) qui sont définis le long de la circonférence extérieure du bord dudit insert (45) et sont complémentaires à ladite ou auxdites protubérances (30).

2. Prothèse cotyloïdienne (10) pour prothèses d'os iliaque selon la revendication 1, **caractérisée en ce que** ledit dos métallique (15) a une épaisseur comprise entre 0,5 et 2 millimètres.

3. Prothèse cotyloïdienne (10) pour prothèses d'os iliaque selon la revendication 1 ou 2, **caractérisée en ce que** la surface extérieure dudit dos métallique (15) comporte une couche (20) de métal poreux constituée de pointes métalliques vives.

4. Prothèse cotyloïdienne (10) pour prothèses d'os iliaque selon la revendication 3, **caractérisée en ce que** ladite couche (20) de métal poreux a une épaisseur comprise entre 0,2 et 1 millimètre.

5. Prothèse cotyloïdienne (10) pour prothèses d'os iliaque selon la revendication 3 ou 4, **caractérisée en ce que** lesdites pointes ont une forme sensiblement triangulaire et une inclinaison comprise entre 3° et 20°.

6. Prothèse cotyloïdienne (10) pour prothèses d'os iliaque selon une ou plusieurs des revendications précédentes, **caractérisée en ce qu'**elle comporte en outre un système (80) pour coupler ledit insert (45) dans ledit dos métallique (15), ledit système de couplage (80) étant constitué d'un gradin annulaire (40) défini sur la surface intérieure dudit dos métallique (15) et d'une fente annulaire (55) définie sur la surface extérieure dudit insert (45).

7. Prothèse cotyloïdienne (10) pour prothèses d'os iliaque selon une ou plusieurs des revendications précédentes, **caractérisée en ce qu'**elle comporte en outre un ou plusieurs points de couplage (35) pour un ensemble osseux ou un extracteur, ledit ou lesdits points de couplage (35) étant définis sur lesdites protubérances (30).

8. Prothèse cotyloïdienne (10) pour prothèses d'os iliaque selon une ou plusieurs des revendications précédentes, **caractérisée en ce que** ledit dos métallique (15) est constitué d'un alliage de titane du type Ti6A14V.

9. Prothèse cotyloïdienne (10) pour prothèses d'os iliaque selon une ou plusieurs des revendications précédentes, **caractérisée en ce que** ledit insert (45) est en polyéthylène UHMWPE.

10. Prothèse cotyloïdienne (10) pour prothèses d'os iliaque selon la revendication 9, **caractérisée en ce que** ledit insert (45) est en polyéthylène UHMWPE qui est réticulé et/ou a un anti-oxydant ajouté.

11. Prothèse cotyloïdienne (10) pour prothèses d'os iliaque selon la revendication 10, **caractérisée en ce que** ledit anti-oxydant comporte de la vitamine E ou des dérivés de celle-ci.

12. Prothèse cotyloïdienne (10) pour prothèses d'os iliaque selon une ou plusieurs des revendications précédentes, **caractérisée en ce qu'**elle est pré-assemblée en usine.
